# EUROPEAN PATENT APPLICATION

(11) **EP 2 098 505 A1**
(43) Date of publication of application: **09.09.2009**
(21) Application number: 08004147.8
(22) Date of filing: 06.03.2008
(51) Int. Cl.: C07C 409/08

(54) **Oxidation of hydrocarbons**

(71) Applicant: ExxonMobil Chemical Patents Inc., Baytown, TX 77520-2101 (US)
(72) Inventor: The designation of the inventor has not yet been filed
(74) Representative: Dew, Melvyn John

(57) **Abstract**

In a process for oxidizing a hydrocarbon to the corresponding hydroperoxide, alcohol, ketone, carboxylic acid or dicarboxylic acid, a reaction medium comprising a hydrocarbon is contacted with an oxygen-containing gas in the presence of a catalyst comprising a cyclic imide of the general formula (I): 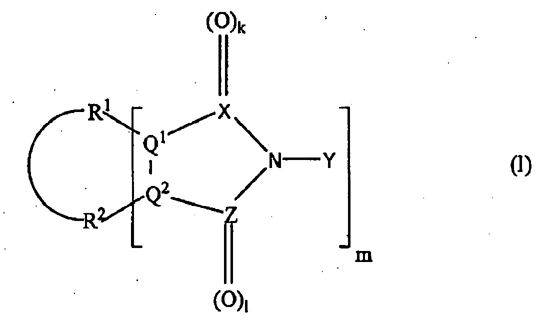 wherein each of R¹ and R² is independently selected from hydrocarbyl and substituted hydrocarbyl radicals having 1 to 20 carbon atoms, or from the groups SO₃H, NH₂, OH and NO₂, or from the atoms H, F, Cl, Br and I provided that R¹ and R² can be linked to one another via a covalent bond; each of Q¹ and Q² is independently selected from C, CH, N, and CR³; each of X and Z is independently selected from C, S, CH₂, N, P and an element of Group 4 of the Periodic Table; Y is O or OH; k is 0, 1, or 2; 1 is 0, 1, or 2; m is 1 to 3; and R³ can be any of the entities listed for R¹. The contacting is conducted under conditions such as to maintain the concentration of both water and organic acids in the reaction medium below 50 ppm.

## Description

### FIELD OF THE INVENTION

The present invention relates to a process for oxidizing hydrocarbons and, in particular, alkylaromatic hydrocarbons to produce for example phenol and substituted phenols.

### BACKGROUND OF THE INVENTION

The oxidation of hydrocarbons is an important reaction in industrial organic chemistry. Thus, for example, the oxidation of cyclohexane is used commercially to produce cyclohexanol and cyclohexanone, which are important precursors in the production of nylon, whereas oxidation of alkylaromatic hydrocarbons is used to produce phenol, a precursor in the production of polycarbonates and epoxy resins.

Oxidation of hydrocarbons can be conducted using well-known oxidizing agents, such as KMnO₄, CrO₃ and HNO₃. However, these oxidizing agents have the disadvantage of being relatively expensive, and moreover their use is accompanied by the production of unwanted coupling products which can represent disposal problems and ecological pollution.

Preferably, therefore, oxidizing agents based on peroxides or N₂O are used. The cheapest oxidizing agent, however, is molecular oxygen, either in pure form or as atmospheric oxygen. However, oxygen itself is usually unsuitable for oxidizing hydrocarbons, since the reactivity of the O₂ molecule, which occurs in the energetically favorable triplet form, is not sufficient.

By using redox metal catalysts it is possible to utilize molecular oxygen for oxidizing organic compounds and hence a great number of industrial processes are based on the metal-catalyzed autooxidation of hydrocarbons. Thus, for example, the oxidation of cyclohexane with O₂ to cyclohexanol and/or cyclohexanone proceeds with the use of cobalt salts. These industrial processes are based on a free-radical chain mechanism, in which the bi-radical oxygen reacts with a hydrocarbon free radical, with formation of a peroxy radical and subsequent chain propagation by abstraction of an H atom from a further hydrocarbon. In addition to metal salts, however, organic molecules can also act as free-radical initiators.

However, it is a disadvantage of these processes that the selectivity decreases very greatly with increasing conversion and therefore the processes must be operated at a very low level of conversion. Thus, for example, the oxidation of cyclohexane to cyclohexanol/cyclohexanone is carried out at a conversion of 10 to 12% so that the selectivity is 80 to 85% ("Industrielle Organische Chemie" [Industrial Organic Chemistry] 1994, 261, VCH-Verlag, D-69451 Weinheim).

An alternative to metal salt catalysts is the use of organic mediators, for example N-hydroxyphthalimide (NHPI). Thus, U.S. Patent Nos. 6,852,893 and 6,720,462 describe methods for oxidizing hydrocarbon substrates by contacting the substrate with an oxygen-containing gas, in which the oxygen content is from 5 to 100% by volume, in the presence of a free radical initiator and a catalyst, typically a N-hydroxycarbodiiinide catalyst, such as N-hydroxyphthalimide (NHPI). The process is conducted at a temperature between 0°C and 500°C and a pressure between atmospheric and 100 bar (100 and 10,000 kPa). The molar ratio of the catalyst to the hydrocarbon substrate can range from 10⁻⁶ mol % to 1 mol %, whereas the molar ratio of free-radical initiator to the catalyst can be 4:1 or less, such as 1:1 to 0.5:1. Suitable substrates that may be oxidized by this process include cumene, cyclohexylbenzene, cyclododecylbenzene and sec-butylbenzene.

U.S. Patent No. 7,038,089 discloses a process for preparing a hydroperoxide from a hydrocarbon selected from a group consisting of primary hydrocarbons, secondary hydrocarbons and mixtures thereof corresponding to said hydroperoxide which comprises conducting the oxidation of said hydrocarbon at a temperature in the range between 130 and 160°C with an oxygen-containing gas in a reaction mixture containing said hydrocarbon and a catalyst comprising a cyclic imide compound and an alkali metal compound. Suitable hydrocarbons are said to include C₄ to C₂₀ tertiary alkanes (e.g., iso-butane, iso-pentane, isohexane, and the like), C₇ to C₂₀ (alkyl) aromatic hydrocarbons with 1 to 6 aromatic rings or C₉ to C₂₀ (cycloalkyl) aromatic hydrocarbons with 1 to 6 aromatic rings (e.g., xylene, cumene, ethylbenzene, diisopropylbenzene, cyclohexylbenzene, tetrahydronaphthalene (tetraline), indane, etc.), and the like. The amount of the cyclic imide compound used may be from 0.0001 to 1%, preferably from 0.0005 to 0.5%, by weight based on the reaction mixture, whereas the amount of the alkali metal compound may be from 0.000005 to 0.01 %, preferably from 0.00001 to 0.005%, by weight based on the reaction mixture

However, while N-hydroxycarbodiimides have shown activity and selectivity for the oxidation of hydrocarbons to products, such as hydroperoxides, they suffer from the problem that they are readily hydrolyzed to non-catalytic species in the presence of the water and organic acids that tend to be generated as common by-products of the oxidation process. Moreover, under oxidation conditions, carbon-carbon bond scission reactions can generate alkyl radicals that can react with oxygen to terminate radical chain propagation and produce undesirable organic acids, such as low molecular weight organic acids eg containing 2, 3 or 4 carbon atoms, such as acetic acid. For example, acetic acid not only facilitates hydrolysis of N-hydroxycarbodiimides, but also catalyzes the exothermic decomposition of hydroperoxide products, which can result in dangerous temperature excursions as well as producing oxidation poisons.

According to the invention, it has now been found that the oxidation of alkylaromatic compounds in the presence of N-hydroxycarbodiimide catalysts can be facilitated by conducting the reaction under conditions such as to maintain the concentration of water and organic acids, especially low molecular weight organic acids, in the reaction medium below 50 ppm. This can be achieved by operating the process at low pressure and low oxygen concentration and by stripping water and organic acids from the reaction products as they are formed.

### SUMMARY OF THE INVENTION

In one aspect, the present invention resides in a process for oxidizing a hydrocarbon to an oxidized hydrocarbon product comprising the corresponding hydroperoxide, alcohol, ketone, carboxylic acid or dicarboxylic acid, the process comprising contacting a reaction medium comprising a hydrocarbon with an oxygen-containing gas in the presence of a catalyst comprising a cyclic imide of the general formula (I): 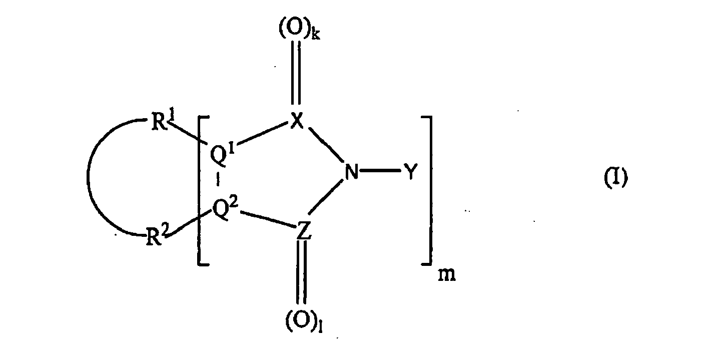 wherein each of R¹ and R² is independently selected from hydrocarbyl and substituted hydrocarbyl radicals having 1 to 20 carbon atoms, or from the groups SO₃H, NH₂, OH and NO₂, or from the atoms H, F, Cl, Br and I, provided that R¹ and R² can be linked to one another via a covalent bond;
each of Q¹ and Q² is independently selected from C, CH, N, and CR³;
each of X and Z is independently selected from C, S, CH₂, N, P and elements of Group 4 of the Periodic Table;
Y is O or OH;
k is 0, 1, or 2;
l is 0, 1, or 2;
m is 1 to 3; and
R³ can be any of the entities (radicals, groups, or atoms) listed for R¹; and
wherein said contacting is conducted under conditions such as to maintain the concentration of water in the reaction medium below 50 ppm by weight and the concentration of organic acids in the reaction medium below 50 ppm by weight.

Conveniently, said hydrocarbon is an alkane or cycloalkane, such as isobutane or cyclohexane.

Alternatively, said hydrocarbon is an alkylaromatic compound of general formula (II): 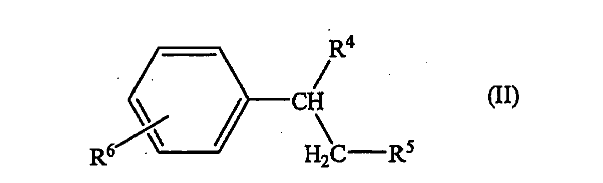 wherein R⁴ and R⁵ each independently represents hydrogen or an alkyl group having from 1 to 4 carbon atoms, provided that R⁴ and R⁵ may be joined to form a cyclic group having from 4 to 10 carbon atoms, said cyclic group being optionally substituted, and R⁶ represents hydrogen, one or more alkyl groups having from 1 to 4 carbon atoms or a cyclohexyl group.

Conveniently, said alkylaromatic compound of general formula (II) is selected from ethyl benzene, cumene, sec-butylbenzene, sec-pentylbenzene, p-methyl-sec-butylbenzene, 1,4-diphenylcyclohexane, sec-hexylbenzene, and cyclohexylbenzene.

Conveniently, said cyclic imide obeys the general formula (III): 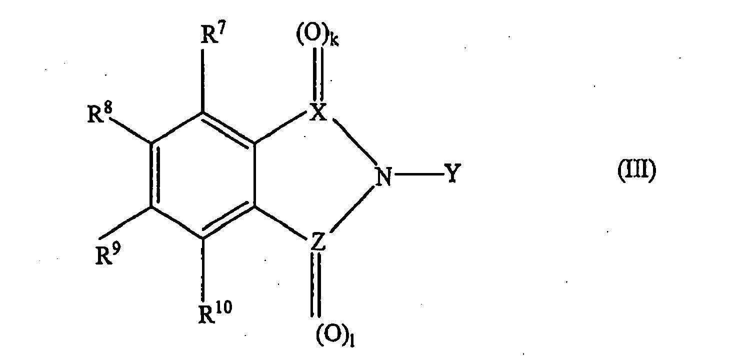 wherein each of R⁷, R⁸, R⁹, and R¹⁰ is independently selected from hydrocarbyl and substituted hydrocarbyl radicals having 1 to 20 carbon atoms, or from the groups SO₃H, NH₂, OH and NO₂, or from the atoms H, F, Cl, Br and I;
each of X and Z is independently selected from C, S, CH₂, N, P and elements of Group 4 of the Periodic Table;
Y is O or OH;
k is 0, 1, or 2; and
l is 0, 1, or 2.

In one embodiment, said cyclic imide comprises N-hydroxyphthalimide (NHPI).

In a further aspect, the present invention resides in a process for producing a phenol, said process comprising:
(a) contacting a reaction medium comprising an alkylaromatic compound of general formula (II): 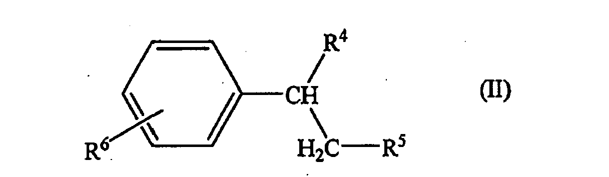 wherein R⁴ and R⁵ each independently represents hydrogen or an alkyl group having from 1 to 4 carbon atoms, provided that R⁴ and R⁵ may be joined to form a cyclic group having from 4 to 10 carbon atoms, said cyclic group being optionally substituted, and R⁶ represents hydrogen, one or more alkyl groups having from 1 to 4 carbon atoms or a cyclohexyl group, with oxygen in the presence of a catalyst comprising a cyclic imide of the general formula (I): 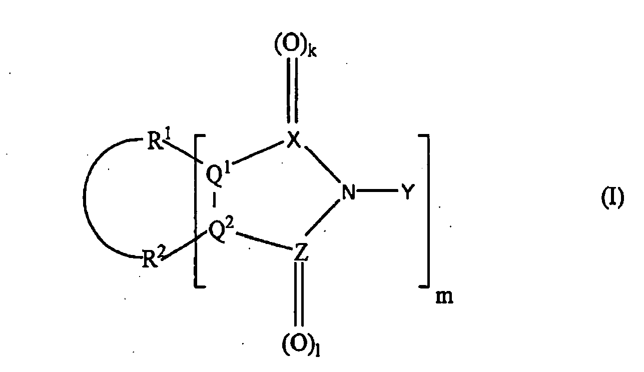 wherein each of R¹ and R² is independently selected from hydrocarbyl and substituted hydrocarbyl radicals having 1 to 20 carbon atoms, or from the groups SO₃H, NH₂, OH and NO₂, or from the atoms H, F, Cl, Br and I, provided that R¹ and R² can be linked to one another via a covalent bond;
   each of Q¹ and Q² is independently selected from C, CH, N, and CR³;
   each of X and Z is independently selected from C, S, CH₂, N, P and elements of Group 4 of the Periodic Table;
   Y is O or OH;
   k is 0, 1, or 2;
   l is 0, 1, or 2;
   m is 1 to 3; and
   R³ can be any of the entities (radicals, groups, or atoms) listed for R¹,
   wherein said contacting is conducted under conditions such as to maintain the concentration of water in the reaction medium below 50 ppm by weight and the concentration of organic acids in the reaction medium below 50 ppm by weight and said contacting produces a hydroperoxide of general formula (IV): 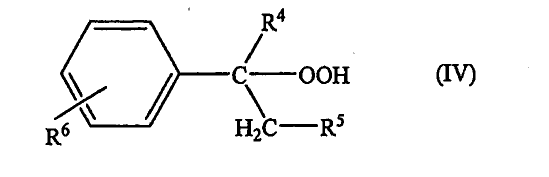 in which R⁴, R⁵ and R⁶ have the same meaning as in formula (II), and
(b) converting the hydroperoxide of formula (IV) into a phenol and an aldehyde or ketone of the general formula R⁴COCH₂R⁵ (V), in which R⁴ and R⁵ have the same meaning as in formula (II).

Conveniently, said contacting is conducted at a pressure below 300 kPa, such as between about 100 kPa and about 200 kPa The temperature of the contacting is conveniently between about 50°C and about 130°C.

Conveniently, said oxygen-containing gas comprises up to 21 volume %, for example from about 0.1 to about 21 volume %, oxygen.

Conveniently, a stripping gas is passed through said reaction medium during said contacting. In one embodiment, said stripping gas is the same as the oxygen-containing gas. In another embodiment, said stripping gas is different from the oxygen-containing gas and is inert to the reaction medium and the cyclic imide catalyst.

### BRIEF DESCRIPTION OF THE DRAWINGS

Figure 1 is a graph plotting acetic acid and NHPI concentration against time on stream (T.O.S.) in the oxidation of sec-butylbenzene (SBB) in the presence of 0.11 wt% NHPI at 690 kPag (100 psig) according to the process of Example 1.

Figure 2 is a graph plotting acetic acid and NHPI concentration against time on stream in the oxidation of SBB in the presence of 0.11 wt% NHPI at 100 kPa (atmospheric pressure) according to the process of Example 2.

Figure 3 is a graph comparing the SBB conversion against time on stream for the processes of Examples 1 and 2.

Figure 4 is a graph comparing the sec-butylbenzene hydroperoxide (SBBHP) selectivity against SBB conversion for the processes of Examples 1 and 2.

Figure 5 is a graph plotting NHPI concentration against time on stream in the oxidation of SBB in the presence of 0.05 wt% NHPI at 690 kPag (100 psig) according to the process of Example 3 and at 100 kPa (atmospheric pressure) according to the process of Example 4.

Figure 6 is a graph plotting SBB conversion against time on stream in the oxidation of SBB in the presence of 0.05 wt% NHPI at 690 kPag (100 psig) according to the process of Example 3 and at 100 kPa (atmospheric pressure) according to the process of Example 4.

Figure 7 is a graph plotting SBBHP selectivity against SBB conversion in the oxidation of SBB in the presence of 0.05 wt% NHPI at 690 kPag (100 psig) according to the process of Example 3 and at 100 kPa (atmospheric pressure) according to the process of Example 4.

Figure 8 is a graph plotting the reaction rate (wt%) against stirrer agitation speed at different pressures in the oxidation of sec-butylbenzene (SBB) in the presence of 0.1 wt% NHPI according to the process of Example 5.

Figure 9 is a graph plotting NHPI concentration against time on stream in the oxidation of sec-butylbenzene (SBB) at 100 kPa (atmospheric pressure) and at 690 kPag (100 psig) in the presence of 0.1 wt% NHPI and with the reaction medium being agitated by a stirrer rotating at 1000 rpm according to the process of Example 5.

Figure 10 is a graph plotting SBB conversion against time on stream in the oxidation of sec-butylbenzene (SBB) at 100 kPa (atmospheric pressure) and at 690 kPag (100 psig) in the presence of 0.1 wt% NHPI and with the reaction medium being agitated by a stirrer rotating at 1300 rpm according to the process of Example 5.

Figure 11 is a graph plotting SBBHP selectivity against time on stream in the oxidation of sec-butylbenzene (SBB) at 100 kPa (atmospheric pressure) and at 690 kPa (100 psig) in the presence of 0.1 wt% NHPI and with the reaction medium being agitated by a stirrer rotating at 1300 rpm according to the process of Example 5.

Figure 12 is a graph plotting the conversion of cyclohexylbenzene (CHB) against time on stream in the oxidation of CHB in the presence of 0.1 wt% NHPI at 100 kPa (atmospheric pressure), 345 kPag (50 psig) and 690 kPag (100 psig) and with stirring at 1000 rpm according to the process of Examples 6 to 8.

Figure 13 is a graph comparing cyclohexylbenzene hydroperoxide (CHBHP) selectivity against CHB conversion for the processes of Examples 6 to 8.

Figure 14 is a graph plotting NHPI concentration against pressure after 4 hours reaction time in the oxidation of CHB according to the processes of Examples 6 to 8.

Figure 15 is a graph plotting CHB conversion against time on stream in the oxidation of cyclohexylbenzene (CHB) at 100 kPa (atmospheric pressure) in the presence of 0.1 wt% and 1 wt% NHPI according to the processes of Examples 6 and 9.

Figure 16 is a graph plotting CHBHP selectivity against CHB conversion in the oxidation of cyclohexylbenzene (CHB) at 100 kPa (atmospheric pressure) in the presence of 0.1 wt% and 1 wt% NHPI according to the processes of Examples 6 and 9.

### DETAILED DESCRIPTION OF THE EMBODIMENTS

The terms "group", "radical", and "substituent" are used interchangeably in this document. For purposes of this disclosure, "hydrocarbyl radical" is defined to be a radical, which contains hydrogen atoms and up to 20 carbon atoms and which may be linear, branched, or cyclic, and when cyclic, aromatic or non-aromatic. "Substituted hydrocarbyl radicals" are radicals in which at least one hydrogen atom in a hydrocarbyl radical has been substituted with at least one functional group or where at least one non-hydrocarbon atom or group has been inserted within the hydrocarbyl radical. Conveniently, each of R¹ and R² is independently selected from aliphatic alkoxy or aromatic alkoxy radicals, carboxy radicals, alkoxy-carbonyl radicals and hydrocarbon radicals, each of which radicals has 1 to 20 carbon atoms.

The present invention provides a process for oxidizing a hydrocarbon to the corresponding hydroperoxide, alcohol, ketone, carboxylic acid or dicarboxylic acid. The process comprises contacting a reaction medium comprising a hydrocarbon with an oxygen-containing gas in the presence of a catalyst comprising a cyclic imide of the general formula (I): 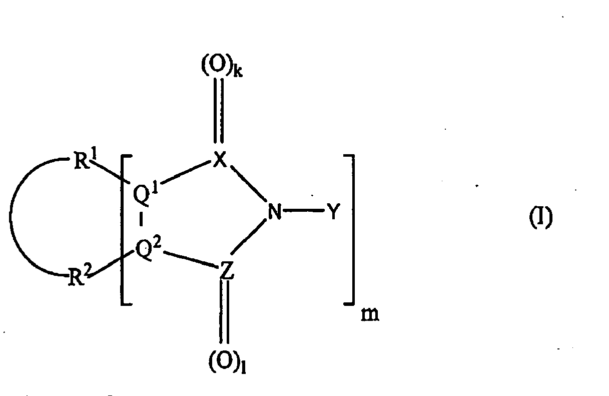 wherein each of R¹ and R² is independently selected from hydrocarbyl and substituted hydrocarbyl radicals having 1 to 20 carbon atoms, or the groups SO₃H, NH₂, OH and NO₂, or the atoms H, F, Cl, Br and I, provided that R¹ and R² can be linked to one another via a covalent bond; each of Q¹ and Q² is independently selected from C, CH, N, and CR³; each of X and Z is independently selected from C, S, CH₂, N, P and elements of Group 4 of the Periodic Table; Y is O or OH; k is 0, 1, or 2; 1 is 0, 1, or 2; m is 1 to 3; and R³ can be any of the entities (radicals, groups, or atoms) listed for R¹. The contacting is conducted under conditions such as to maintain the concentration of water in the reaction medium below 50 ppm and the concentration of organic acids in the reaction medium below 50 ppm so that hydrolysis of the cyclic imide catalyst is minimized. Preferably the weight concentration of water and organic acids other than target acids in the reaction medium is each independently maintained, by selection of process conditions, at below 40 wppm, for example in the range 10 to 40 wppm such as 20 to 30 wppm.

As used herein, the new numbering scheme for the Periodic Table Groups are employed as disclosed in Chemical and Engineering News, 63(5), 27 (1985).

### Hydrocarbon Feed

Using the present process a wide group of substituted or unsubstituted saturated or unsaturated hydrocarbons, such as alkanes, cycloalkanes, alkenes, cycloalkenes, and aromatics, can be selectively oxidized. In particular, however, the process has utility in the selective oxidation of isobutane to tertiary butyl hydroperoxide and tertiary butanol, the selective oxidation of cyclohexane to cyclohexanol and cyclohexanone and the selective oxidation of alkylaromatic compounds of the general formula (II) to the corresponding hydroperoxides: 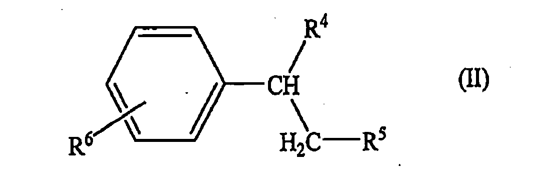 in which R⁴ and R⁵ each independently represents hydrogen or an alkyl group having from 1 to 4 carbon atoms, provided that R⁴ and R⁵ may be joined to form a cyclic group having from 4 to 10 carbon atoms, said cyclic group being optionally substituted, and R⁶ represents hydrogen, one or more alkyl groups having from 1 to 4 carbon atoms or a cyclohexyl group. In an embodiment, R⁴ and R⁵ are joined to form a cyclic group having from 4 to 10 carbon atoms, conveniently a cyclohexyl group, substituted with one or more alkyl group having from 1 to 4 carbon atoms or with one or more phenyl groups. Examples of suitable alkylaromatic compounds are ethyl benzene, cumene, sec-butylbenzene, sec-pentylbenzene, p-methyl-sec-butylbenzene, 1,4-diphenylcyclohexane, sec-hexylbenzene, and cyclohexylbenzene, with sec-butylbenzene and cyclohexylbenzene being preferred. It will also be understood that in the case where R⁴ and R⁵ are joined to form a cyclic group, the number of carbons forming the cyclic ring is from 4 to 10. However, that ring may itself carry one or more substituents, such as one or more alkyl groups having from 1 to 4 carbon atoms or one or more phenyl groups, as in the case of 1,4- diphenylcyclohexane.

In one practical embodiment, the alkylaromatic compound of general formula (II) is sec-butylbenzene and is produced by alkylating benzene with at least one C₄ alkylating agent under alkylation conditions and in the presence of a heterogeneous catalyst, such as zeolite beta or more preferably at least one molecular sieve of the MCM-22 family (as defined below). The alkylation conditions conveniently include a temperature of from about 60°C to about 260°C, for example between about 100°C and about 200°CThe alkylation pressure is conveniently 7000 kPa or less, for example from about 1000 to about 3500 kPa. The alkylation is conveniently carried out at a weight hourly space velocity (WHSV) based on C₄ alkylating agent of between about 0.1 and about 50 hr⁻¹, for example between about 1 and about 10 hr⁻¹.

The C₄ alkylating agent conveniently comprises at least one linear butene, namely butene-1, butene-2 or a mixture thereof. The alkylating agent can also be an olefinic C₄ hydrocarbon mixture containing linear butenes, such as can be obtained by steam cracking of ethane, propane, butane, LPG and light naphthas, catalytic cracking of naphthas and other refinery feedstocks and by conversion of oxygenates, such as methanol, to lower olefins. For example, the following C₄ hydrocarbon mixtures are generally available in any refinery employing steam cracking to produce olefins and are suitable for use as the C₄ alkylating agent: a crude steam cracked butene stream, Raffinate-1 (the product of remaining after solvent extraction or hydrogenation to remove butadiene from the crude steam cracked butene stream) and Raffinate-2 (the product remaining after removal of butadiene and isobutene from the crude steam cracked butene stream).

In a further practical embodiment, the alkylaromatic compound of general formula (II) is cyclohexylbenzene and is produced by contacting benzene with hydrogen in the presence of a heterogeneous bifunctional catalyst which comprises at least one metal having hydrogenation activity, typically selected from the group consisting of palladium, ruthenium, nickel and cobalt, and a crystalline inorganic oxide material having alkylation activity, typically at least one molecular sieve of the MCM-22 family (as defined below). The contacting step is conveniently conducted at a temperature of about 50°C to about 350°C. The contacting pressure may be, for example, from about 100 to about 7000 kPa. The benzene to hydrogen molar ratio in the contacting step is preferably from about 0.01 to about 100. The WHSV during the contacting step is preferably in the range of about 0.01 to about 100.

The term "MCM-22 family material" (or "material of the MCM-22 family" or "molecular sieve of the MCM-22 family" or "MCM-22 family zeolite"), as used herein, includes one or more of:
- molecular sieves made from a common first degree crystalline building block unit cell, which unit cell has the MWW framework topology. (A unit cell is a spatial arrangement of atoms which if tiled in three-dimensional space describes the crystal structure. Such crystal structures are discussed in the "Atlas of Zeolite Framework Types", Fifth edition, 2001, the entire content of which is incorporated as reference);
- molecular sieves made from a common second degree building block, being a 2-dimensional tiling of such MWW framework topology unit cells, forming a monolayer of one unit cell thickness, preferably one c-unit cell thickness;
- molecular sieves made from common second degree building blocks, being layers of one or more than one unit cell thickness, wherein the layer of more than one unit cell thickness is made from stacking, packing, or binding at least two monolayers of one unit cell thickness. The stacking of such second degree building blocks can be in a regular fashion, an irregular fashion, a random fashion, or any combination thereof; and
- molecular sieves made by any regular or random 2-dimensional or 3-dimensional combination of unit cells having the MWW framework topology.

Molecular sieves of the MCM-22 family include those molecular sieves having an X-ray diffraction pattern including d-spacing maxima at 12.4±0.25, 6.9±0.15, 3.57±0.07 and 3.42±0.07 Angstrom. The X-ray diffraction data used to characterize the material are obtained by standard techniques such as using the K-alpha doublet of copper as incident radiation and a diffractometer equipped with a scintillation counter and associated computer as the collection system.

Materials of the MCM-22 family include MCM-22 (described in U.S. Patent No. 4,954,325), PSH-3 (described in U.S. Patent No. 4,439,409), SSZ-25 (described in U.S. Patent No. 4,826,667), ERB-1 (described in European Patent No. 0293032), ITQ-1 (described in U.S. Patent No 6,077,498), ITQ-2 (described in International Patent Publication No. WO97/17290), MCM-36 (described in U.S. Patent No. 5,250,277), MCM-49 (described in U.S. Patent No. 5,236,575), MCM-56 (described in U.S. Patent No. 5,362,697), UZM-8 (described in U.S. Patent No. 6,756,030), and mixtures thereof. Molecular sieves of the MCM-22 family are preferred as the alkylation catalyst since they have been found to be highly selective to the production of sec-butylbenzene, as compared with the other butylbenzene isomers. Preferably, the molecular sieve is selected from (a) MCM-49, (b) MCM-56 and (c) isotypes of MCM-49 and MCM-56, such as ITQ-2.

### Hydrocarbon Oxidation

The oxidation step in the present process is effected by contacting the hydrocarbon substrate with an oxygen-containing gas in the presence of a catalyst comprising a cyclic imide of the general formula (I): 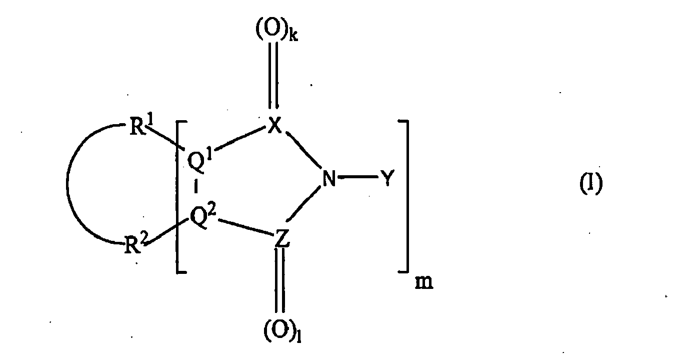 wherein each of R¹ and R² is independently selected from hydrocarbyl and substituted hydrocarbyl radicals having 1 to 20 carbon atoms, or the groups SO₃H, NH₂, OH and NO₂, or the atoms H, F, Cl, Br and I provided that R¹ and R² can be linked to one another via a covalent bond; each of Q¹ and Q² is independently selected from C, CH, N, and CR³; each of X and Z is independently selected from C, S, CH2, N, P and elements of Group 4 of the Periodic Table; Y is O or OH; k is 0, 1, or 2; 1 is 0, 1, or 2; m is 1 to 3, and R³ can be any of the entities (radicals, groups, or atoms) listed for R¹. Conveniently, each of R¹ and R² is independently selected from aliphatic alkoxy or aromatic alkoxy radicals, carboxyl radicals, alkoxy-carbonyl radicals and hydrocarbon radicals, each of which radicals has 1 to 20 carbon atoms.

Generally, the cyclic imide employed as the oxidation catalyst obeys the general formula 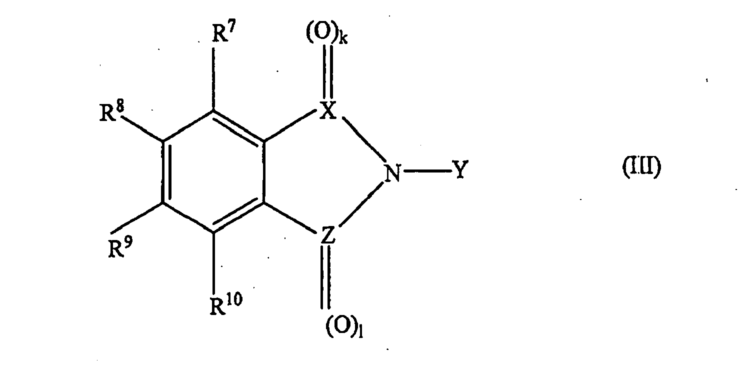 wherein each of R⁷, R⁸, R⁹, and R¹⁰ is independently selected from hydrocarbyl and substituted hydrocarbyl radicals having 1 to 20 carbon atoms, or the groups SO₃H, NH₂, OH and NO₂, or the atoms H, F, Cl, Br and I; each of X and Z is independently selected from C, S, CH₂, N, P and elements of Group 4 of the Periodic Table; Y is O or OH; k is 0, 1, or 2, and 1 is 0, 1, or 2. Conveniently, each of R⁷, R⁸, R⁹, and R¹⁰ is independently selected from aliphatic alkoxy or aromatic alkoxy radicals, carboxyl radicals, alkoxy-carbonyl radicals and hydrocarbon radicals, each of which radicals has 1 to 20 carbon atoms.

In one practical embodiment, the cyclic imide catalyst comprises N-hydroxyphthalimide (NHPI).

The contacting of the hydrocarbon substrate with an oxygen-containing gas in the presence of the cyclic imide catalyst is conducted under conditions that are effective not only to effect the desired oxidation of the substrate but also to maintain the concentration of both water and organic acids (e.g., acetic or formic acid) in the reaction medium below 50 ppm. Such conditions typically include conducting the oxidation at relatively low pressure, such as below 300 kPa, for example between about 100 kPa and about 200 kPa. Moreover, although the oxidation can be conducted over a broad oxygen concentration range between 0.1 and 100%, it is preferred to operate at relatively low oxygen concentration, such as no more than 21 volume %, for example from about 0.1 to about 21 volume %, generally from about 1 to about 10 volume %, oxygen in the oxygen-containing gas. In addition, maintaining the desired low levels of water and organic acids, is facilitated by passing a stripping gas through the reaction medium during the oxidation step. In one embodiment, the stripping gas is the same as the oxygen-containing gas. In another embodiment, the stripping gas is different from the oxygen-containing gas and is inert to the reaction medium and the cyclic imide catalyst. Suitable stripping gases include inert gases, such as helium and argon.

An additional advantage of operating the oxidation process at low pressure and low oxygen concentration and by stripping water and organic acids, from the reaction medium is that light hydroperoxide (e.g., ethyl or methyl hydroperoxide), light ketones (e.g., methyl ethyl ketone), light aldehydes (e.g., acetaldehyde) and light alcohols (e.g., ethanol) are removed from the reaction products as they are formed. Thus light hydroperoxides are hazardous and pose a safety concern if their concentration in the liquid product becomes too high. Also, light hydroperoxides, alcohols, aldehydes and ketones are precursors for the formation of organic acids and water so that removing these species from the oxidation medium improves the oxidation reaction rate and selectivity and the stability of the cyclic imide catalyst. In fact, data shows that when conducting oxidation of sec-butylbenzene with NHPI at 690 kPag (100 psig), more than 99 mol% of these light species and water remain in the reactor, whereas at atmospheric pressure, more than 95 mol% of these species are removed from the oxidation reactor.

A suitable temperature for the oxidation step is between about 20°C and about 300°C, more particularly between about 50°C and about 130°C.

### Oxidation Product

The product of the present oxidation process depends on the nature of the hydrocarbon substrate being oxidized but in general is a hydroperoxide, alcohol, ketone, carboxylic acid or dicarboxylic acid, especially a hydroperoxide.

For example, when the hydrocarbon substrate is isobutane, the oxidation product comprises tertiary butyl hydroperoxide (which is useful as an oxidation reagent and in the production of propylene oxide) and tertiary butanol (which is useful as a gasoline additive).

When the hydrocarbon substrate is cyclohexane, the oxidation product comprises cyclohexyl hydroperoxide, cyclohexanol and cyclohexanone. Cyclohexyl hydroperoxide is readily decomposed to additional cyclohexanol and cyclohexanone, either thermally or with the assistance of a catalyst. Cyclohexanol can be oxidized with aqueous nitric acid to produce adipic acid, which is a precursor in the synthesis of Nylon 6,6, whereas cyclohexanone can be converted to cyclohexanoxime which undergoes acid-catalyzed rearrangement to produce caprolactam, a precursor in the synthesis of Nylon 6.

Where the hydrocarbon substrate is an alkylaromatic compound of the general formula (II), the product of the oxidation reaction includes a hydroperoxide of general formula (IV): 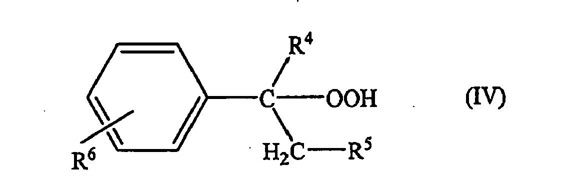 in which R⁴, R⁵ and R⁶ have the same meaning as in formula (II). Preferably, the hydroperoxide is sec-butylbenzene hydroperoxide or cyclohexylbenzene hydroperoxide. This hydroperoxide can then be converted by acid cleavage to phenol or a substituted phenol and an aldehyde or ketone of the general formula R⁴COCH₂R⁵ (V), in which R⁴ and R⁵ have the same meaning as in formula (II).

The cleavage reaction is conveniently effected by contacting the hydroperoxide with a catalyst in the liquid phase at a temperature of about 20°C to about 150°C, such as about 40°C to about 120°C, and/or a pressure of about 50 to about 2500 kPa, such as about 100 to about 1000 kPa and/or a liquid hourly space velocity (LHSV) based on the hydroperoxide of about 0.1 to about 1000 hr⁻¹, preferably about 1 to about 50 hr⁻¹. The hydroperoxide is preferably diluted in an organic solvent inert to the cleavage reaction, such as methyl ethyl ketone, phenol, cyclohexylbenzene, cyclohexanone and sec-butylbenzene, to assist in heat removal. The cleavage reaction is conveniently conducted in a catalytic distillation unit.

The catalyst employed in the cleavage step can be a homogeneous catalyst or a heterogeneous catalyst.

Suitable homogeneous cleavage catalysts include sulfuric acid, perchloric acid, phosphoric acid, hydrochloric acid and p-toluenesulfonic acid. Ferric chloride, boron trifluoride, sulfur dioxide and sulfur trioxide are also effective homogeneous cleavage catalysts. The preferred homogeneous cleavage catalyst is sulfuric acid

A suitable heterogeneous catalyst for use in the cleavage of sec-butylbenzene hydroperoxide includes a smectite clay, such as an acidic montmorillonite silica-alumina clay, as described in U.S. Patent No. 4,870,217 (Texaco), the entire disclosure of which is incorporated herein by reference.

The invention will now be more particularly described with reference to the following non-limiting Examples.

### Example 1

150 gm of sec-butylbenzene (SBB) supplied by TCI America and 0.16 gm (0.11 wt%) of N-hydroxyphthalimide (NHPI) were weighed into a Parr reactor fitted with a stirrer, thermocouple, gas inlet, sampling port and a condenser containing a Dean Stark trap for water removal. The reactor and contents were stirred at 700 rpm and sparged with nitrogen at a flow rate of 250 cc/minute for 5 minutes. The reactor was then pressurized with nitrogen to 690 kPag (100 psig) while maintained under a nitrogen sparge and was then heated to 125°C. When the reaction temperature was reached, the gas was switched from nitrogen to air and the reactor was sparged with air at 250 cc/minute for 4 hours. Samples were taken hourly and the NHPI, acetic acid and reactant concentrations of each sample were measured by gas chromatography for conversion and selectivity and HPLC (High pressure liquid chromatography) for NHPI concentration measurements. For water analysis Karl Fischer analysis was used. After 4 hours, the gas was switched back to nitrogen and the heat was turned off. When the reactor had cooled, it was depressurized and the contents removed.

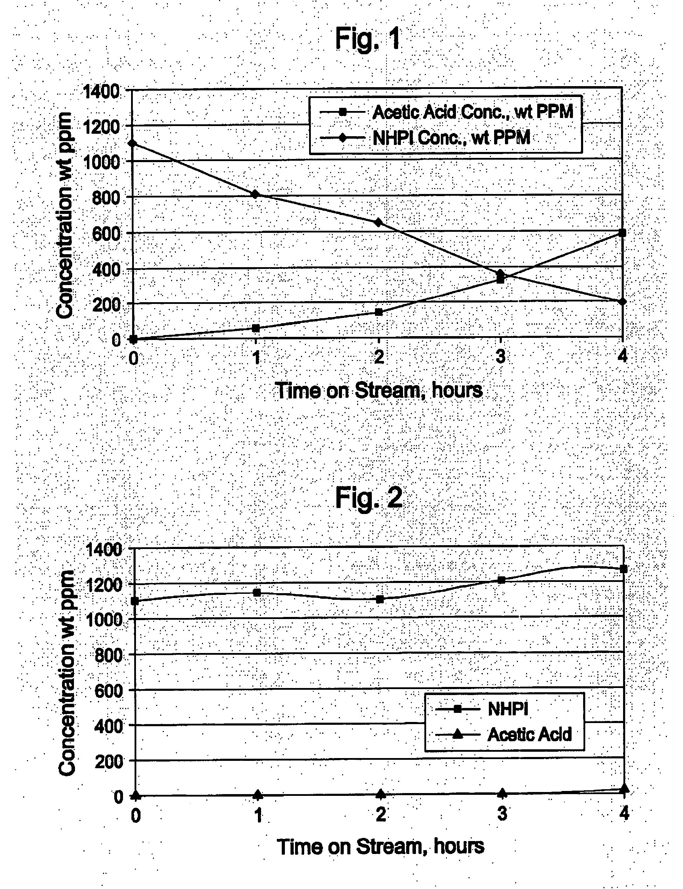

The results are shown in Figure 1, from which it will be seen that the NHPI concentration dropped from 1100 wppm at the beginning of the test to 185 wppm over 4 hours, during which time the acetic acid concentration built up from zero to about 600 wppm. Over the same time the ethyl hydroperoxide concentration built up to about 2000 wppm.

### Example 2

The process of Example 1 was repeated but with the test being run at atmospheric pressure (100 kPa) and the results are shown in Figure 2. It will be seen that, at atmospheric pressure (100 kPa), acetic acid and other light acids were formed at very low concentration and were completely removed from the reaction mixture, making them unavailable to catalyze the hydrolysis of the NHPI. In fact for the first 3 hours of the test, the acetic acid and the ethyl hydroperoxide levels were below the detection limits of the gas chromatographic analysis, rising to only 30 and 34 ppm for acetic acid and ethyl hydroperoxide respectively (in contrast with the 600 and 2000 ppm obtained in Example 1), by the end of the 4 hour run time. In addition to stripping acetic acid from the reaction mixture, at low pressure the air flowing through the reactor stripped out the majority of the water also generated as a byproduct of the oxidation process, to a level below 50 wppm.

The NHPI concentration during the test of Example 2 is also shown in Figure 2, from which it will be seen that the concentration of NHPI started out at 1100 ppm and drifted up slowly during the test as other volatile components were stripped from the reaction mixture. Thus, at low pressure, with most of the acetic acid and water removed to levels below 50 wppm, the NHPI catalyst in the reaction vessel was protected from hydrolysis and remained close to its starting concentration and available for the oxidation reaction.

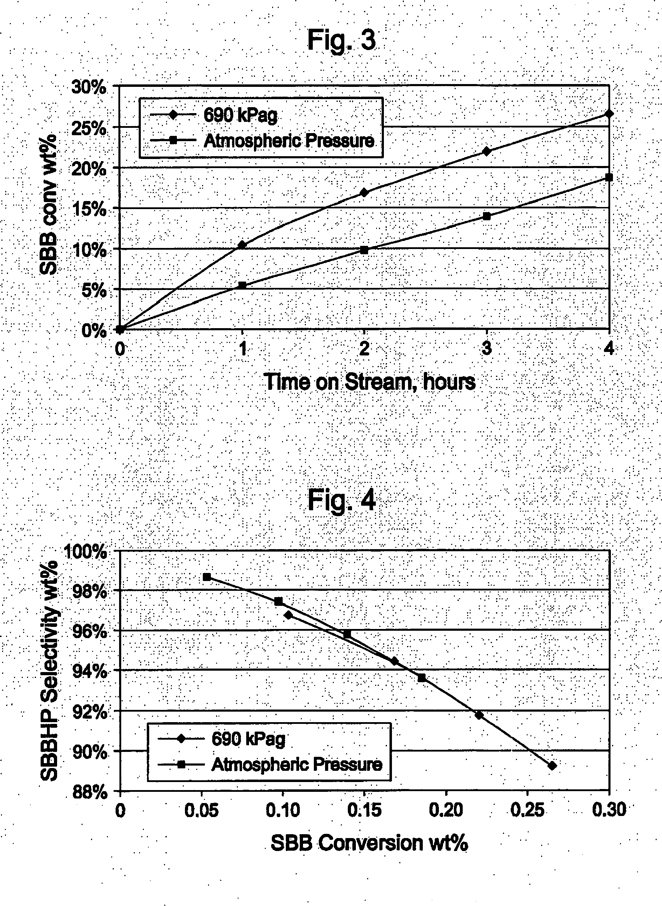

The SBB conversion and the selectivity to sec-butylbenzene hydroperoxide (SBBHP) for Examples 1 and 2 are compared in Figures 3 and 4. It will be seen from Figure 3 that the SBB conversion was adversely affected by the lower pressure used in Example 2, decreasing from 26 wt% after 4 hours in Example 1 to only 18 wt% in Example 2. However, as can be seen from Figure 4, the SBBHP selectivity versus SBB conversion response was very similar at both 690 kPag (100 psig) and atmospheric pressure, indicating that the SBBHP selectivity was set by the SBB conversion selected and was not a function of pressure at these reaction conditions.

### Examples 3 and 4

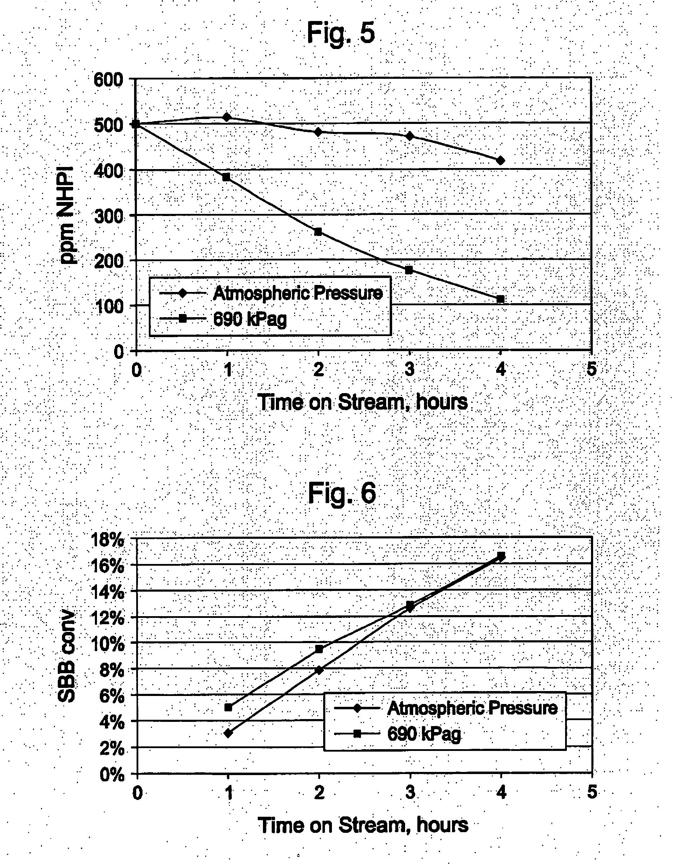

The processes of Examples 1 and 2 were repeated but with the initial NHPI concentration being decreased to 0.05 wt% and the results are shown in Figures 5 to 7.

Figure 5 plots the NHPI concentration against time on stream and shows that the concentration dropped from 500 ppm to 100 ppm over the 4 hours of the test at 690 kPag (100 psig) in Example 3, but only dropped to 420 ppm over 4 hours at atmospheric pressure (100 kPa) in Example 4.

Figure 6 plots the SBB conversion against time on stream and shows that the conversion in both Examples 3 and 4 was essentially the same at about 17 wt% after the 4 hours of the test.

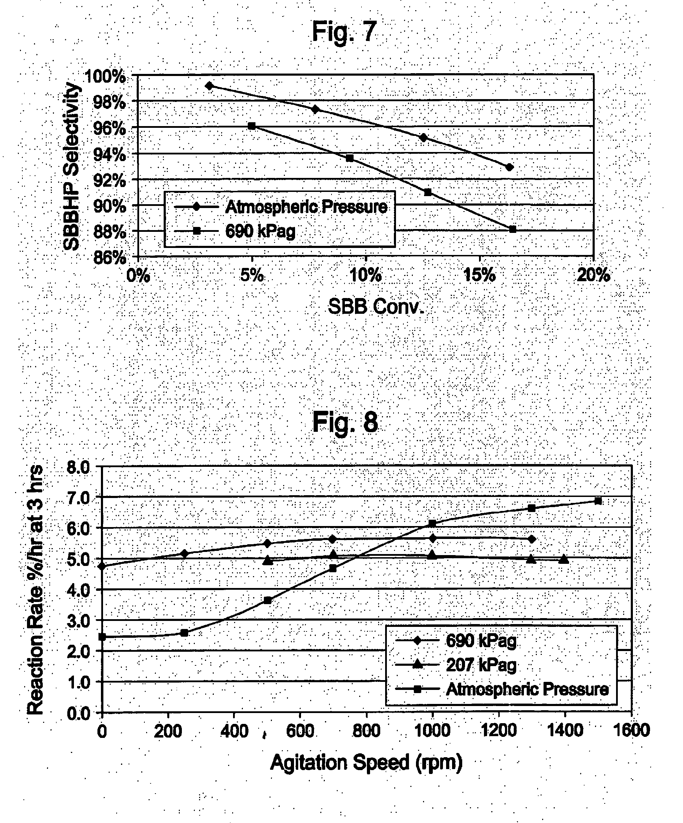

Figure 7 plots the SBBHP selectivity against the SBB conversion and shows that the SBBHP selectivity was significantly better at atmospheric pressure (100 kPa) than at 690 kPag (100 psig) over the entire range of SBB conversions tested.

### Example 5

As shown in Figure 3, the SBB conversion in Example 2 was adversely affected by the lower pressure employed, indicating that oxygen mass transfer (oxygen concentration in the liquid phase) is the limiting step in the oxidation reaction. One approach for improving the oxygen concentration in the liquid phase is to increase the rate of agitation of the reaction medium, by increasing the agitation speed of a stirrer in contact with the reaction medium. Thus in Example 5, the effect of stirrer agitation speed was evaluated at different pressures, namely at 690 kPag (100 psig), 207 kPag (30 psig) and atmospheric pressure (100 kPa). In particular, the process of Example 1 was repeated but with the reactor and contents being stirred at various rates between 0 and 1500 rpm at each of the pressures tested. The results are summarized in Figures 8 to 11.

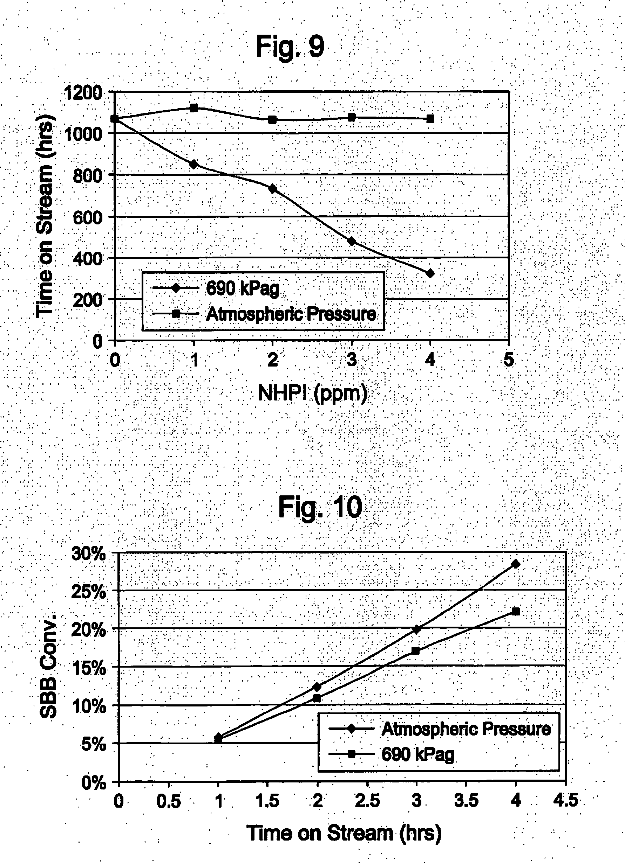

Figure 8 plots the reaction rate (wt%) against agitation speed at different pressures and shows that the reaction rate at high pressure, 207 kPag and 690 kPag (30 and 100 psig), is almost not affected by the agitation speed. However, at atmospheric pressure, the reaction rate is dramatically affected by the agitation speed. This is presumably because undesirable product mixture components (water and organic acid, especially low molecular weight organic acid such as acetic acid) are removed to levels below 50 wppm at atmospheric pressure and the NHPI stability is improved (Figure 9). At the same time the oxygen concentration in the liquid phase is increased by the high speed agitation.

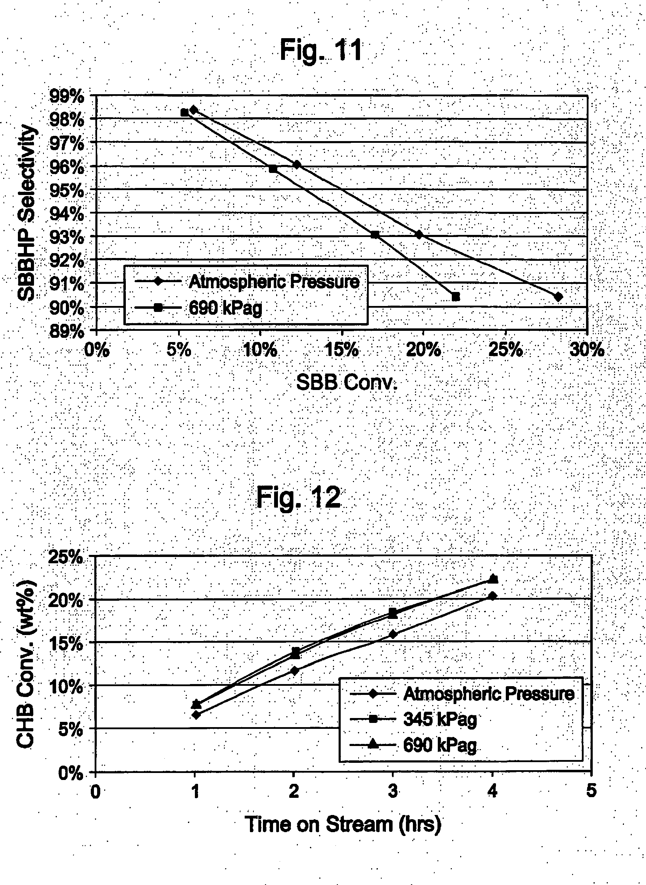

Figures 10 and 11 show that, at high speed agitation and atmospheric pressure, not only is the NHPI stability improved but also the SBB conversion and SBBHP selectivity are increased.

### Example 6

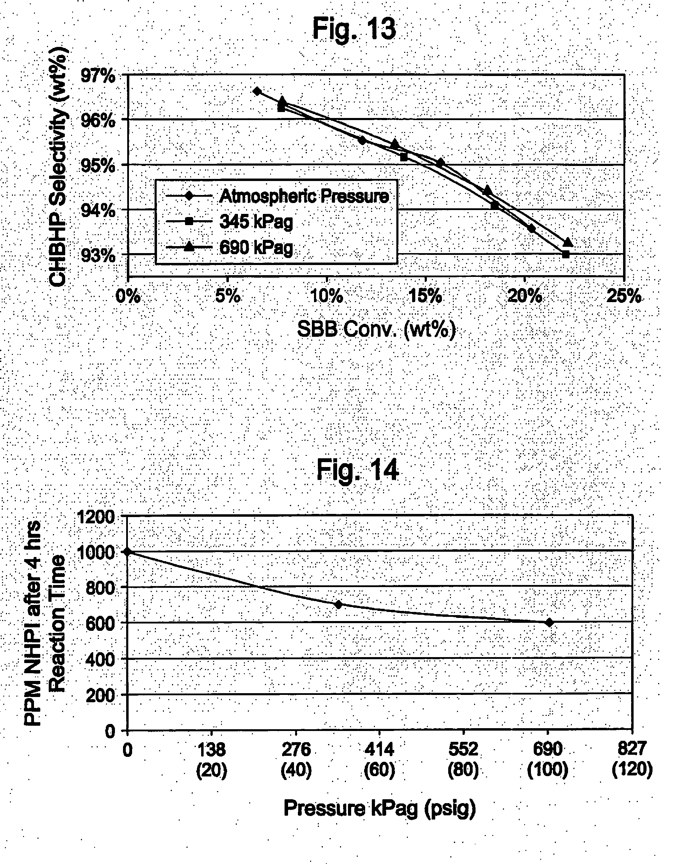

The process of Example 1 was repeated but with the test being run with 150 gm of cyclohexyl benzene (CHB) supplied by TCI America and 0.16 gm (0.11 wt%) of N-hydroxyphthalimide (NHPI) being weighed into a Parr reactor fitted with a stirrer, thermocouple, gas inlet, sampling port and a condenser containing a Dean Stark trap for water removal. The reactor and contents were stirred at 1000 rpm and sparged with nitrogen at a flow rate of 250 cc/minute for 5 minutes. The reactor was then pressurized with nitrogen to 690 kPag (100 psig)while maintained under a nitrogen sparge and was then heated to 115°C. When the reaction temperature was reached, the gas was switched from nitrogen to air and the reactor was sparged with air at 250 cc/minute for 4 hours. Samples were taken hourly and the NHPI and the reaction concentrations of each sample were measured by gas chromatography for conversion and selectivities and HPLC (high pressure liquid chromatography) for NHPI concentrations measurements. After 4 hours, the gas was switched back to nitrogen and the heat was turned off. When the reactor had cooled, it was depressurized and the contents removed. The results are shown in Figure 12, 13 and 14.

### Example 7

The process of Example 6 was repeated but with the test being run at a pressure of 345 kPag (50 psig). Again the results are shown in Figure 12,13 and 14.

### Example 8

The process of Example 6 was repeated but with the test being run at atmospheric pressure (100 kPa). Again the results are shown in Figure 12, 13 and 14.

It will be seen from Figure 12, 13 and 14 that the CHB conversion and the selectivity to the hydroperoxide were not affected by the pressure decrease between Examples 6 to 8. However, the NHPI concentration was affected by the pressure employed in the test. Thus, at atmospheric pressure, the NHPI concentration remains the same over the 4 hours of the test, but at 345 and 690 kPag (50 and 100 psig) the NHPI concentration dropped from 1100 ppm at the beginning of the test to 700 and 600 ppm respectively at the end of the test.

### Example 9

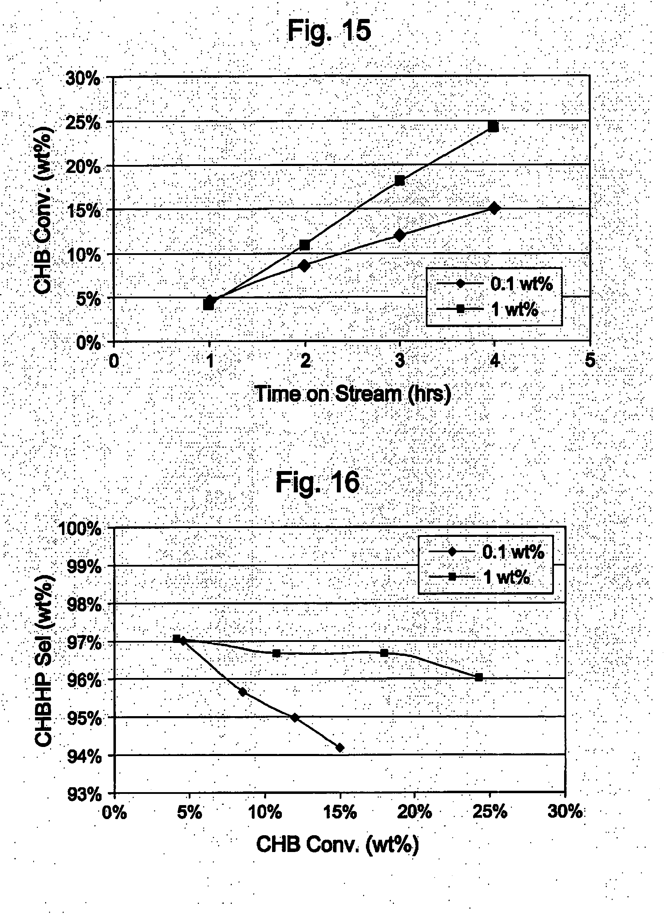

The process of Example 6 was repeated but with the test being run with 1 wt% NHPI at atmospheric pressure and at 110°C The results are shown in Figure 15 and 16, from which it will be seen that the conversion and selectivity were dramatically improved by the presence of 1 wt% NHPI. In addition, the NHPI concentration remained the same, with no NHPI decomposition being observed using HPLC analysis.

While the present invention has been described and illustrated by reference to particular embodiments, those of ordinary skill in the art will appreciate that the invention lends itself to variations not necessarily illustrated herein. For this reason, then, reference should be made solely to the appended claims for purposes of determining the true scope of the present invention.

## Claims

1. A process for oxidizing a hydrocarbon to an oxidized hydrocarbon product comprising the corresponding hydroperoxide, alcohol, ketone, carboxylic acid or dicarboxylic acid, the process comprising contacting a reaction medium comprising a hydrocarbon with an oxygen-containing gas in the presence of a catalyst comprising a cyclic imide of the general formula (I): 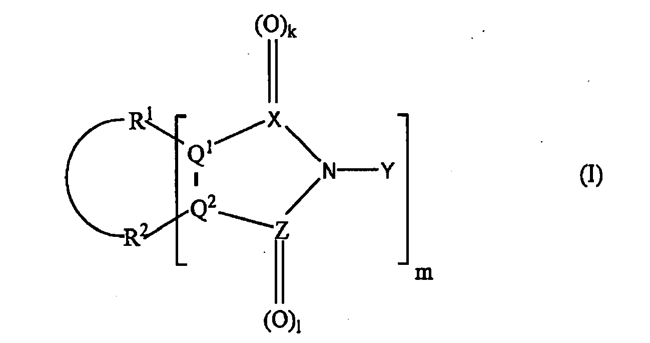 wherein each of R¹ and R² is independently selected from hydrocarbyl and substituted hydrocarbyl radicals having 1 to 20 carbon atoms, or from the groups SO₃H, NH₂, OH and NO₂ or from the atoms H, F, Cl, Br and I, provided that R¹ and R² can be linked to one another via a covalent bond;
each of Q¹ and Q² is independently selected from C, CH, N, and CR³;
each of X and Z is independently selected from C, S, CH₂, N, P and elements of Group 4 of the Periodic Table;
Y is O or OH;
k is 0, 1, or 2;
l is 0, 1, or 2;
m is 1 to 3; and
R³ can be any of the entities listed for R¹; and
wherein said contacting is conducted under conditions such as to maintain the concentration of water in the reaction medium below 50 ppm by weight and the concentration of organic acids in the reaction medium below 50 ppm by weight.

2. The process of claim 1, wherein the cyclic imide obeys the general formula (III): 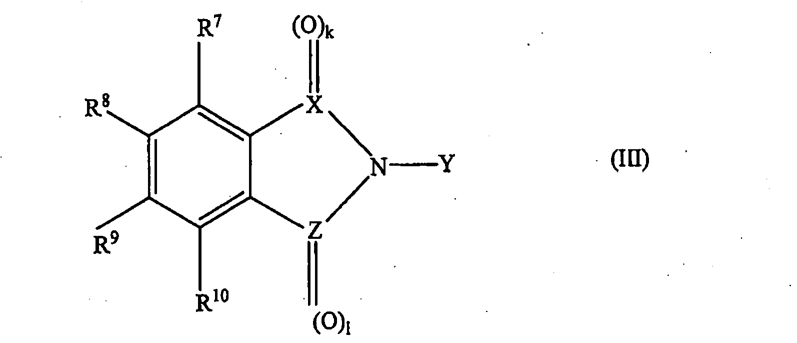 wherein each of R⁷, R⁸, R⁹, and R¹⁰ is independently selected from hydrocarbyl and substituted hydrocarbyl radicals having 1 to 20 carbon atoms, or from the groups SO₃H, NH₂, OH and NO₂ or from the atoms H, F, Cl, Br and I;
each of X and Z is independently selected from C, S, CH₂, N, P and elements of Group 4 of the Periodic Table;
Y is O or OH;
k is 0, 1, or 2; and
l is 0,1, or 2.

3. The process of claim 1 or 2, wherein the cyclic imide comprises N-hydroxyphthalimide.

4. The process of claim 3 wherein the pressure is between 100 kPa and 200 kPa.

5. The process of any preceding claim, wherein the contacting is conducted at a temperature of between 50°C and 130°C.

6. The process of any preceding claim, wherein the oxygen-containing gas comprises up to 21 volume % oxygen.

7. The process of any preceding claim, wherein a stripping gas is passed through the reaction medium during the contacting.

8. The process of claim 7, wherein the stripping gas is different from the oxygen-containing gas and is inert to the reaction medium and the cyclic imide catalyst.

9. The process of any preceding claim, wherein the hydrocarbon comprises cyclohexane, the oxidized hydrocarbon product comprises cyclohexanol or cyclohexanone and the process further comprises converting the cyclohexanol to adipic acid or the cyclohexanone to caprolactam.

10. The process of any one of claims 1 to 8, wherein the hydrocarbon comprises an alkylaromatic compound of general formula (II): 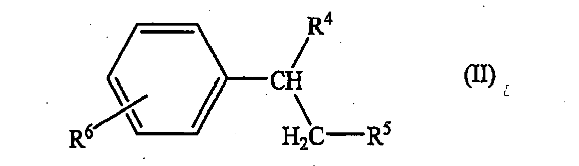 wherein R⁴ and R⁵ each independently represents hydrogen or an alkyl group having from 1 to 4 carbon atoms, provided that R⁴ and R⁵ may be joined to form a cyclic group having from 4 to 10 carbon atoms, said cyclic group being optionally substituted, and R⁶ represents hydrogen, one or more alkyl groups having from 1 to 4 carbon atoms or a cyclohexyl group, and wherein the oxidized hydrocarbon product comprises a hydroperoxide of general formula (IV): 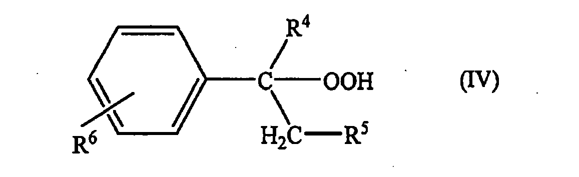 in which R⁴, R⁵ and R⁶ have the same meaning as in formula (II).

11. The process of claim 10, wherein the alkylaromatic compound of general formula (II) is selected from ethyl benzene, cumene, sec-butylbenzene, sec-pentylbenzene, p-methyl-sec-butylbenzene, 1,4-diphenylcyclohexane, sec-hexylbenzene, and cyclohexylbenzene.

12. The process of claim 10 or 11 and further comprising converting the hydroperoxide of formula (IV) from the organic phase into a phenol and an aldehyde or ketone of the general formula R⁴COCH₂R⁵ (V), in which R⁴ and R⁵ have the same meaning as in formula (II).

13. The process of claim 12 and further comprising converting the phenol produced into bisphenol A.
